# EUROPÄISCHE PATENTSCHRIFT

(11) **EP 4 044 901 B1**
(45) Veröffentlichungstag und Bekanntmachung des Hinweises auf die Patenterteilung: **26.03.2025**
(21) Anmeldenummer: 20780905.4
(22) Anmeldetag: 24.09.2020
(51) Int. Cl.: A61B 5/00, A61B 5/103, A61G 7/057

(54) **VORRICHTUNG ZUR LAGEBESTIMMUNG EINES BECKENS EINER PERSON**
DEVICE FOR DETERMINING THE POSITION OF A PERSON'S PELVIS
DISPOSITIF DE DÉTERMINATION DE LA POSITION DU BASSIN D'UNE PERSONNE

(30) Priorität: 16.10.2019 AT 508962019
(43) Veröffentlichungstag der Anmeldung: 24.08.2022
(73) Patentinhaber: Sanlas Holding GmbH, 8010 Graz (AT)
(72) Erfinder: ZENZMAIER, Cornelia, 8301 Laßnitzhöhe (AT)
(74) Vertreter: Röggla, Harald
(86) Internationale Anmeldenummer: PCT/AT2020/060344
(87) Internationale Veröffentlichungsnummer: WO 2021/072462

(56) Entgegenhaltungen:
- EP-A1- 3 011 896
- JP-A- 2003 111 646
- JP-A- 2005 261 508
- US-A1- 2005 137 462

## Beschreibung

Die vorliegende Erfindung betrifft eine Vorrichtung nach Anspruch 1.

Das Becken gilt als Stellungsregler für die neutrale Ausrichtung der funktionellen Wirbelsäule und der unteren Extremitäten. Obwohl das Becken alters- und geschlechtsspezifische Unterschiede hinsichtlich der Längen- und Winkelverhältnisse aufweist, kann jedoch eine allgemein gültige einheitliche Beschreibung für dessen lagebedingte Belastung erfolgen. Bei Sitzsystemen wird das Becken der Person im Allgemeinen von der Sitzfläche und der Rückenlehne des Sitzsystems gestützt. Die Sitzbeinhöcker dienen hierbei zur Aufnahme des Oberkörpergewichts und können bei einer ergonomisch richtigen im Wesentlichen aufrechten Sitzposition der Person hohen Sitzdruck aufnehmen, wohingegen das Steißbein und das Kreuzbein bei dieser Sitzposition im Wesentlichen unbelastet bleiben. In horizontaler Rückenlage stützt die Wirbelsäule den Großteil der Oberkörpergewichtskraft ab und das Körperabschnittsgewicht im Bereich des Beckens der Person lastet auf dem Kreuzbein, wobei der von der Gewichtskraft der Person ausgeübte Auflagedruck am Kreuzbein in dieser Lage im Wesentlichen maximal ist und das Steißbein im Wesentlichen unbelastet bleibt. Bei einer ergonomisch richtigen Bauchlage übernimmt das Schambein der Person den maximalen Anteil jenes Körperabschnittsgewichts.

Personen sind sich ihrer eigenen individuellen Körperhaltung im Stehen, Sitzen und Liegen oft nicht bewusst und nehmen Positionen ein, die einseitige schmerzhafte Muskelkontrakturen verursachen und langfristig bei Fehlbelastung der Wirbelsäulen- und Beckenstruktur zu Abnutzungsprozessen, auch Bandscheibenvorfällen, führen können. Insbesondere langes Sitzen erfordert permanente Haltearbeit der Muskulatur oft in Fehlhaltung und ohne ausreichende Ausgleichsbewegungen, wodurch muskuläre Dysbalancen hervorgerufen und vielfältige körperliche Beschwerden begünstigt werden. Durch das Einnehmen einer solchen ergonomisch falschen Position, insbesondere einer falschen Sitzposition, kann das Steiß- oder Kreuzbein mit Teilen der Gewichtskraft der Person belastet werden, wodurch es zu einem Missverhältnis der Belastungen des Beckens und der Wirbelsäulenstruktur, insbesondere im Übergangsbereich des Beckens in die Wirbelsäule, den Iliosakralgelenken, kommen kann. Außerdem kann es hierdurch zu Stauchungen und Verpressungen der gesamten Wirbelsäule, insbesondere im Bereich der Lendenwirbelsäule kommen, da die Person aufgrund von oftmals weichen Untergrundstrukturen und verminderter Muskelspannung zur Wahrung einer individuell ergonomisch korrekten Sitzposition in passiver Sitzhaltung über einen längeren Zeitraum hinweg, zu einer krummen Körperhaltung verleitet wird, dessen Ursache in der lagebedingten Beckenstellung zu finden ist. Besonders nachteilhaft ist dies für pflegebedürftige Personen und/oder Personen in Rollstühlen, da diese ihre falsche Sitzposition aufgrund ihrer physischen Einschränkung nicht selbst verbessern können, sondern auf Hilfe Dritter, z. B. von Krankenpflegepersonal, angewiesen sind. Da die zuvor genannten Personen oftmals keine Schmerzen, beispielsweise aufgrund einer Querschnittslähmung oder einer neurodegenerativen Erkrankung spüren, oder keine Schmerzen äußern können, sind diese besonders gefährdet, bleibende Schäden durch das Einnehmen einer falschen Sitzposition davonzutragen.

Um dies zu verhindern und einer möglichen Fehlbelastung der Wirbelsäule, und den damit verbundenen Risiken körperlicher Langzeitschäden entgegenzuwirken, sind dem Stand der Technik Sitzsysteme mit Sitzflächen bekannt, welche eine Aussparung in der Sitzfläche im Bereich des Steißbeines aufweisen. Diese Sitzsysteme entlasten zwar das Steißbein beziehungsweise die Steißbeinregion, wirken einer falschen Sitzposition beziehungsweise einer für die entsprechende Lage falschen Beckenrotation, insbesondere um die Horizontalachse, jedoch nicht entgegen, da beispielsweise Krümmungsveränderungen der Wirbelsäule, die sich auf die Lage des Beckens auswirken können, nicht erfasst werden. Andere aus dem Stand der Technik bekannte Sitzsysteme, z. B. moderne Autositze, verwenden fluidgefüllte und mittels einer Pumpvorrichtung aufpumpbare Druckelemente mit Sensoren im Bereich der Lendenwirbelsäule und/oder im Bereich der Sitzbeinhöcker, um eine ergonomisch richtige Sitzposition zu erzielen.

Ein solcher Autositz ist in US20170086588A1 offenbart, welcher ein Luftkammersystem mit zwei lateral beabstandete und in die Sitzfläche eines Sitzes integrierte Luftkammerelemente umfasst, die bei ungleichmäßigem Sitzen das Becken der sitzenden Person durch das Ausbalancieren deren Sitzbeinhöcker durch Zu- oder Abführen von Luft mittels eines Kompressors, positioniert. In den Luftkammerelementen sind Kontaktdrucksensoren integriert, welche Sitzdrücke, die die Sitzbeinhöcker des Benutzers auf die Sitzfläche ausüben, erfassen. Ein Controller wertet die Sitzdrücke aus und steuert den Kompressor entsprechend der Auswertung an.

Von Nachteil ist, dass bei diesem Sitzsystem die jeweilige individuelle Lage des Beckens nicht exakt erfasst wird, sondern lediglich die von den Sitzbeinhöckern auf die Luftkammerelemente übertragenen Sitzdrücke bestimmt werden. Damit beschränkt sich dieses Sitzsystem auf einen horizontalen Ausgleich der Sitzbeinhöcker durch eine Rotation des Beckens um die Sagittalachse. Bei einer falschen, durch die Person selbst eingenommenen Sitzposition kann ein zu stark aufgepumptes Luftkammerelement beispielsweise Krümmungsänderungen der Wirbelsäule verursachen, was zu einer anhaltenden Überstreckung der Lendenwirbelsäule aufgrund der lagebedingten falschen Beckenrotation um die Horizontalachse führen kann. Ein zu schwach aufgepumptes Druckelement kann hingegen zu einem Rundrücken mit ursächlicher posteriorer Beckenkippung führen, was die bereits oben genannten Nachteile, insbesondere die Belastung des Steiß- oder Kreuzbeins mit einem Teil der Gewichtskraft der Person in einer aufrechten Sitzhaltung, mit sich bringt. Diese muskuläre Überstreckung oder Stauchung der Wirbelsäule mit einseitiger Fehlbelastung der Bandscheiben aufgrund einer lagebedingten falschen Beckenstellung kann von dem Sitzsystem des oben genannten Standes der Technik nicht erfasst werden, wodurch körperliche Folgeschäden auch in den an die Lendenwirbelsäule anschließenden Bereichen der Brust- und Halswirbelsäule nicht verhinderbar sind.

Aus dem Stand der Technik (EP 3 011 896 A1, EP 1 093 755 A1, JP 2003 111646 A, US 2005/137462 A1) sind weiters Systeme zur Überwachung der Sitzhaltung einer Person mittels in einem Stuhl eingebrachten Drucksensoren bzw. Vorrichtungen zur Messung der Auflagedrücke einer Person im Liegen bekannt. Solche Systeme bzw. Vorrichtungen sind jedoch zur Lagebestimmung des Beckens einer Person nicht geeignet.

Die frühere Patentanmeldung A 50386/2019 des Anmelders, welche zum Zeitpunkt dieser Patentanmeldung noch nicht veröffentlicht ist, offenbart eine Vorrichtung zur Körperpositionierung einer Person mit Stellelementen und Sensoren, die auf einem Sitzelement und auf einem Rückenelement angeordnet sind. In dieser früheren Patentanmeldung ist der Abrollvorgang der Person während deren Überführung von einer aufrechten in eine horizontale Liegeposition erwähnt, wobei das Becken der Person durch lineare Stellbewegungen durch Rotationen um die Sagittal-, Horizontal- und Longitudinalachse positioniert wird. Eine Lage des Beckens wird nur indirekt bestimmt, indem das Becken der Person positioniert wird, sobald ein vorgegebener Sitz- und/oder Auflagedruckunterschied erreicht wird. Hierdurch kann die Lage des Beckens nicht exakt bestimmt werden. Um einer falschen Sitzposition entgegenzuwirken ist es daher notwendig, die Lage des Beckens der Person, d. h. die Neigung und die Rotation des Beckens um die Körperachsen der Person, exakt und für jede Position der Person auf einem Sitz- oder Liegesystem zu bestimmen.

Die Aufgabe der vorliegenden Erfindung ist es, eine Vorrichtung zur Lagebestimmung des Beckens einer Person bereitzustellen, welche in ein bereits bestehendes Sitzsystem oder Liegesystem integrierbar ist und welche die oben genannten Nachteile des Standes der Technik vermeidet.

Die vorliegende Erfindung löst die gestellten Aufgaben durch Bereitstellen einer Vorrichtung zur Lagebestimmung eines Beckens einer Person mit den Merkmalen des Anspruchs 1.

In einem weiteren Aspekt stellt die Erfindung ein Computerprogrammprodukt bereit, welches Befehle umfasst, die bei der Ausführung des Programms durch die Rechnereinheit diese veranlassen, die Schritte von Anspruch 1 auszuführen.

In einem weiteren Aspekt stellt die Erfindung einen computerlesbaren Datenträger bereit, auf dem das Computerprogrammprodukt gespeichert ist.

Vorteilhafte Ausgestaltungen der Erfindung ergeben sich aus den Unteransprüchen, der Beschreibung und den Zeichnungen.

Die gegenständliche Erfindung stellt eine Vorrichtung bereit, wobei die Auswerteeinheit dazu ausgebildet ist, die folgenden Schritte auszuführen: das Empfangen von Messsignalen von den Druckübertragungselementen und Auswerten dieser, das Bestimmen einer ersten charakteristischen Druckverteilung, bei der das erste Druckübertragungselement während einer Rotation des Beckens der Person um deren Horizontalachse erstmals mit zumindest einem Teil des Sitzdruckes des Steißbeins oder das erste Druckübertragungselement mit zumindest einem Teil des Auflagedruckes des Schambeins beaufschlagt wird, und das Bestimmen einer zweiten charakteristischen Druckverteilung, bei der der auf das erste Druckübertragungselement ausgeübte Sitzdruck des Steißbeins einen sprunghaften Abfall aufweist und der auf das zweite Druckübertragungselement ausgeübte Auflagedruck des Kreuzbeins einen sprunghaften Anstieg aufweist.

Durch das Bestimmen der oben gennannten charakteristischen Druckverteilungen kann die Lage des Beckens bezüglich der Horizontalachse der Person exakt ermittelt werden. Hierfür ist jene Person, deren Lage des Beckens zu bestimmen ist, auf dem Sitz- oder Liegesystem, im Fall von pflegebedürftigen Personen durch Dritte, entsprechend der Anordnung der Druckübertragungselemente auf dem Sitzsystem vorzupositionieren. Nach dem Ermitteln der charakteristischen Druckverteilung kann durch den Stand der Technik bekannte Mittel und Methoden das Becken der Person anschließend so positioniert werden, dass eine lagebedingte ergonomisch korrekte Stellung des Beckens zur weiteren neutralen Ausrichtungsmöglichkeit der Wirbelsäule für jede, insbesondere um die Horizontalachse der Person gekippte Lage an dem Sitzsystem beibehalten werden kann. Die oben erwähnten körperlichen Beschwerden wie einseitige schmerzhafte Muskelkontrakturen oder Abnützungserscheinungen an der Wirbelsäule können hierdurch vermindert beziehungsweise sogar gänzlich vermieden werden, da die individuell ergonomisch korrekte Ausrichtung der funktionellen Wirbelsäule in Bezug zum Becken erfolgt. Außerdem kann die auf einem Sitzsystem sitzende Person hierdurch entsprechend ihrer Anatomie belastungsgerecht auf dem Sitzsystem positioniert und gelagert werden, wobei diese neutrale Ausrichtung der funktionellen Wirbelsäule auch in Rotationen um die Sagittal- oder Longitudinalachse beibehalten werden kann.

Durch die Möglichkeit, die vorliegende Erfindung in ein bereits bestehendes Sitzsystem zu integrieren oder als Sitzauflage auf einen Stuhl oder Bett zu verwenden, ergeben sich eine Vielzahl von Anwendungsmöglichkeiten, wobei die Vorrichtung nicht auf folgende Beispiele beschränkt ist. Die Vorrichtung könnte beispielsweise in folgende Einrichtungen integriert werden: in Bürosessel, Rollstühle, Fahrzeugsitze, Kinderrückhaltesysteme, Trainingsgeräte, Behandlungsliegen, Matratzen und/oder im Rehabilitations- und Therapiebereich verwendet werden, insbesondere in Stehbetten, Stehbrettern und/oder Operationsbetten. Die Integration der gegenständlichen Erfindung in Operationsbetten ist vor allem für in Bauchlage oder in Rückenlage durchgeführte Eingriffe sinnvoll, da diese oft mit geneigten Operationsbetten durchgeführt werden und es hierdurch zu einer unerwünschten Verlagerung der auf dem Operationsbett befindlichen Person kommen kann beziehungsweise zu einer Fehlpositionierung des Beckens in Bezug zur Ausrichtung der Wirbelsäule, bei der die Wirbelsäule, insbesondere im Lumbalbereich in eine gestauchte oder gestreckte Lage gezwungen wird. Ein Eingriff bei einem derart positionierten Becken kann zu einer Vielzahl von Problemen führen. Durch die gegenständliche Erfindung kann eine von der neutralen lagebedingten Stellung des Beckens abweichende Stellung durch Erfassen von Sitz- und Auflagedruckänderungen zwischen dem ersten und dem zweiten Druckübertragungselement erkannt und dieser Stellung durch die oben erwähnten Mittel und Methoden entgegengewirkt werden. Dadurch kann die neutrale lagebedingte Stellung des Beckens neuerlich hergestellt und beibehalten werden. Im Zuge einer Operation auf zumeist flach ausgeführten Operationsbetten kann ein Eingriff somit in bestmöglicher Körperpositionierung durchgeführt und mögliche Operationsrisiken, beziehungsweise die zum Teil sehr schmerzhaften Folgen einer stundenlangen Fehlpositionierung, insbesondere durch eine auf die Beckenstellung abgestimmte Stützung der Lendenwirbelsäule, minimiert oder sogar ausgeschlossen werden.

Gemäß einer bevorzugten Ausführungsform der Vorrichtung zur Lagebestimmung des Beckens einer Person, sind das erste Druckübertragungselement und das zweite Druckübertragungselement im Wesentlichen in einem stumpfen Winkel zueinander angeordnet. Hierdurch ist der Vorteil erhalten, dass die erste und die zweite charakteristische Druckverteilung auch während einer Änderung der Neigung des Oberkörpers der Person, d. h. während einem Abrollvorgang deren Beckens von einer im Wesentlichen aufrechten Sitzposition in eine im Wesentlichen horizontalen Liegeposition, bestimmt werden können.

Gemäß einer bevorzugten Ausführungsform der Vorrichtung zur Lagebestimmung des Beckens einer Person ist die Rechnereinheit ferner dazu ausgebildet, die von den Druckübertragungselementen an die Rechnereinheit übertragenen Messsignale periodisch in einem vorgegebenen Zeitintervall auszuwerten. Hierdurch kann die Lage des Beckens kontinuierlich, beispielsweise sekündlich, überwacht werden und eine falsche Lagerung eines Pflegebedürftigen durch z. B. ein ungeschultes Personal vermieden werden.

Gemäß einer bevorzugten Ausführungsform der Vorrichtung zur Lagebestimmung des Beckens einer Person ist die Auswerteeinheit eine eine Membran umfassende und mit den Druckübertragungselementen über Kanäle verbundene Membraneinheit, welche einen an der Membran angeordneten Sensor und eine Rechnereinheit aufweist, welche zum Auswerten eines von dem Sensor ausgegebenen Sensorsignals ausgebildet ist. Die von dem Becken der Person auf die Druckübertragungselemente ausgeübten Sitz- und Auflagedrücke verursachen in den Druckübertragungselementen eine Umverteilung des darin enthaltenen Fluides. Durch die Membraneinheit kann diese Umverteilung in ein von der Rechnereinheit auswertbares Sensorsignal, vorzugsweise ein elektrisches Sensorsignal, umgewandelt werden. Hierdurch ist der Vorteil enthalten, dass die Vorrichtung rasch und zuverlässig auf eine Lageänderung oder eine von der neutralen lagebedingten Stellung abweichende Stellung des Beckens reagieren kann.

Gemäß einer weiteren Ausführungsform der Vorrichtung zur Lagebestimmung des Beckens einer Person umfasst die Vorrichtung ein drittes Druckübertragungselement und ein viertes Druckübertragungselement, welche jeweils neben dem zweiten Druckübertragungselement angeordnet und zur Aufnahme der von den Beckenkämmen der Person ausgeübten Auflagedrücke ausgebildet sind. Hierdurch ist es möglich, die Lage des Beckens der Person bei einer Rotation dessen um die Longitudinalachse zu ermitteln, was insbesondere vorteilhaft ist für die Erfassung von Mikro- und Makrolagerungen in der Dekubitusprophylaxe. Bei diesen Lagerungen wird eine Verschiebung des zumindest abschnittsweisen Körperschwerpunktes erzielt, wodurch bestimmte Körperareale druckentlastet werden. Eine bekannte Form der Lagerung ist die 30-Grad-Schräglage, die, ausgehend von einer im Wesentlichen horizontalen Rückenlage, im Allgemeinen mit Hilfe von zwei großen Kissen erreicht wird und die rechte oder linke Gesäß- beziehungsweise Körperhälfte belastet. In einem Sitz- oder Liegesystem des oben genannten Typs integriert, kann die Vorrichtung die oben genannte Verschiebung des Körperabschnittsschwerpunktes im pelvinen Bereich erfassen und die Lage des Beckens in einer Seitenlage und, bei einer zusätzlichen Rotation der Person um deren Longitudinalachse, auch in einer Bauchlage der Person bestimmen.

Gemäß einer weiteren Ausführungsform der Vorrichtung zur Lagebestimmung des Beckens einer Person umfasst die Vorrichtung ein fünftes Druckübertragungselement und ein sechstes Druckübertragungselement, welche jeweils neben dem ersten Druckübertragungselement angeordnet und zum Aufnehmen der von den Sitzbeinhöckern der Person ausgeübten Sitzdrücke ausgebildet sind. Die Sitzbeinhöcker der Person sind in einem vorbestimmten Abstand voneinander entfernt und definieren ein Sitzbeinhöckerniveau, welches, wenn die Person eine ergonomisch richtige Sitzposition eingenommen hat, horizontal um die Sagittalachse ausgeglichen ist. Aus einer Vielzahl von Gründen, beispielsweise durch langes Sitzen oder aufgrund von körperlichen Beeinträchtigungen, kann einer der zwei Sitzbeinhöcker stärker belastet werden, was zu einem unausgeglichenen Sitzbeinhöckerniveau führt und auf Dauer schädlich für die Becken- und Wirbelsäulenstruktur, insbesondere sind hier die Iliosakralgelenke zu nennen, ist. Durch das zusätzliche fünfte und sechste Druckübertragungselement kann die Vorrichtung eine asymmetrische Sitzdruckbelastung der Sitzbeinhöcker detektieren, und, durch Bilden einer Differenz der Sitzdrücke des fünften und des sechsten Druckübertragungselements durch die Rechnereinheit, auch die Lage des Beckens in Bezug auf die Sagittalachse bestimmen. Damit kann die Vorrichtung die Lage des Beckens bezüglich aller drei Körperebenen der Person bestimmen und somit ein ergonomisch korrekter Belastungszustand exakt und allgemein gültig für jede Positionierung der Person, d. h. Sitz- und Liegeposition, an einem Sitz- oder Liegesystem charakterisiert werden.

Durch diese Ausführungsform der Erfindung kann die Vorrichtung, neben der Lage des Beckens in Bezug auf die Horizontalachse der Person, auch die Lage des Beckens in Bezug auf die Sagittalachse und Longitudinalachse bestimmt werden. Die Information über die Lage des Beckens kann anschließend von einem dem Stand der Technik bekannten Sitzsystem genützt werden, um die genannte asymmetrische Sitz- und Auflagedruckbelastung zu korrigieren beziehungsweise Mikro- oder Makrolagerungen durchzuführen.

Gemäß einer weiteren Ausführungsform der Vorrichtung zur Lagebestimmung des Beckens einer Person umfasst die Vorrichtung ein Array mit einer Vielzahl von Druckübertragungselementen, welches dazu ausgebildet ist, die Sitz- und Auflagedrücke des Beckens der Person in jeder Position der Person auf der Vorrichtung zu detektieren. Hierdurch ist der Vorteil enthalten, dass der oben erwähnte Schritt des Vorpositionierens der Person auf einem Sitzsystem entfallen kann, da bei dieser Ausführungsform neben der Lage auch der Ort des Beckens auf dem Sitzsystem bestimmbar ist.

Gemäß einer bevorzugten Ausführungsform der Vorrichtung zur Lagebestimmung des Beckens einer Person ist der Sensor ausgewählt aus der Gruppe bestehend aus mechanischen, elektrischen, pneumatischen oder hydraulischen Sensoren. So ist es möglich, die von dem Becken der Person ausgeübten Sitz- und/oder Auflagedrücke durch eine Vielzahl von Sensoren zu erfassen. Beispielsweise können mechanische Sensoren wie Dehnungsmessstreifen oder aber auch Beschleunigungssensoren zur Erfassung der Sitz- und/oder Auflagedrücke verwendet werden. Bei der Verwendung von Dehnungsmesstreifen kann die Notwendigkeit einer Membraneinheit entfallen, wobei das von dem Sensor ausgegebene Sensorsignal hierdurch direkt durch die Auswerteeinheit, z. B. einem Computer, ausgewertet werden kann. Der Begriff der Sensoren ist nicht auf die oben genannten Beispiele beschränkt.

Gemäß einer bevorzugten Ausführungsform der Vorrichtung zur Lagebestimmung des Beckens einer Person umfasst die Vorrichtung Warnmittel, welche die Person warnt, wenn die erste charakteristische Druckverteilung und/oder die zweite charakteristische Druckverteilung bestimmt wurde. Das Warnmittel ist dabei ausgewählt aus der Gruppe bestehend aus optischen, akustischen oder haptischen Warnmitteln. Hierdurch wird ermöglicht, dass Personen, die eine ergonomisch falsche Sitz- oder Liegeposition eingenommen haben, auf ihre falsche Sitz- oder Liegeposition aufmerksam gemacht werden können und somit mehr Bewusstsein für ihre Körperhaltung entwickeln. Das regelmäßige Erinnern an eine falsche Sitz- oder Liegeposition kann somit helfen, körperliche Schäden der eingangs erwähnten Art vorzubeugen. Im Fall von Pflegebedürftigen hat der Einsatz eines Warnmittels den Vorteil, dass Pflegekräfte auf eine ergonomisch schlechte und/oder falsche Lagerung der pflegebedürftigen Personen aufmerksam gemacht werden können, wodurch sich das Intervall der Kontrolle durch eine Pflegekraft ausdehnen lässt.

Die Erfindung wird nun unter Bezugnahme auf die Zeichnungen anhand von nicht einschränkenden Ausführungsbeispielen näher erläutert.
Fig. 1a zeigt eine schematische Darstellung der Erfindung mit einer auf einem verstellbaren Sitzsystem befindlichen Person in einer ergonomisch richtigen aufrechten Sitzposition.
Fig. 1b zeigt eine schematische Darstellung der Erfindung einer auf dem verstellbaren Sitzsystem in geneigter Lage befindlichen Person, deren Steißbein einen Sitzdruck auf ein erstes Druckelement ausübt.
Fig. 1c zeigt eine schematische Darstellung der Erfindung mit einer auf dem verstellbaren Sitzsystem in geneigter Lage befindlichen Person, deren Kreuzbein einen Auflagedruck auf ein zweites Druckelement ausübt.
Fig. 1d zeigt eine schematische Darstellung der Erfindung mit einer auf dem verstellbaren Sitzsystem in horizontaler Liegeposition befindlichen Person, deren Kreuzbein einen Auflagedruck auf das zweite Druckelement ausübt.
Fig. 2 zeigt ein Diagramm, in welchem Druckverläufe der während einer Rotation des Beckens der Person um die Horizontalachse auf das verstellbare Sitzsystem ausgeübten Sitz- und Auflagedrücke und Markierungen dargestellt sind, die eine erste charakteristische Druckverteilung und eine zweite charakteristische Druckverteilung gemäß Anspruch 1 der Erfindung veranschaulichen.
Fig. 3 zeigt eine bevorzugte Ausführungsform einer Auswerteeinheit.
Fig. 4 zeigt eine perspektivische Ansicht eines Sitzsystems mit daran angeordneten Druckübertragungselementen und der Auswerteeinheit von Fig. 3 gemäß einer bevorzugten Ausführungsform der Erfindung.
Fig. 5 zeigt eine perspektivische Ansicht eines Sitzsystems mit daran angeordneten Druckübertragungselementen und der Auswerteeinheit von Fig. 3 gemäß einer weiteren Ausführungsform der Erfindung.
Fig. 6 zeigt eine perspektivische Ansicht eines Sitzsystems mit daran angeordneten Druckübertragungselementen und der Auswerteeinheit von Fig. 3 gemäß einer weiteren Ausführungsform der Erfindung.

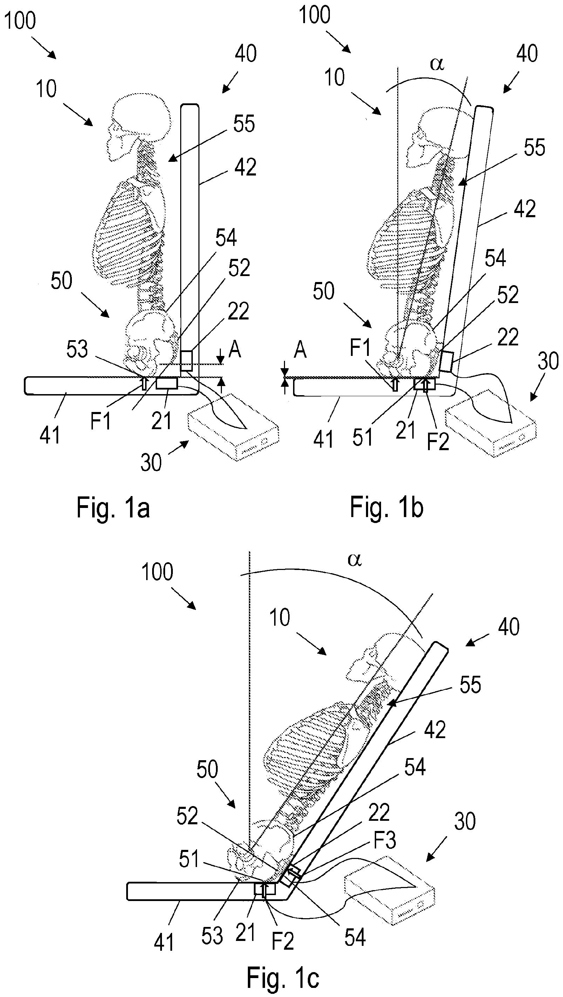

Nachfolgend wird auf die Fig. 1a bis 1d Bezug genommen, die eine Person 10 in einer aufrechten, einer geneigten und in einer horizontal liegenden Position auf einem verstellbaren Sitzsystem 40 mit einem Sitzelement 41 und einem Rückenelement 42 zeigen, in welchem die erfindungsgemäße Vorrichtung 100 integriert dargestellt ist. Die Vorrichtung 100 kann dabei auch in einem Liegesystem, nicht dargestellt, oder in einer Auflage, ebenfalls nicht dargestellt, integriert sein und ist nicht auf das in den Fig. 1a bis 1d dargestellte Sitzsystem 40 beschränkt. Die Vorrichtung 100 ist hierbei dazu ausgebildet, die Person 10 aufzunehmen und umfasst zumindest ein zur Aufnahme eines von dem Steißbein 51 beziehungsweise Schambein der Person 10 ausgeübten Sitzdruckes beziehungsweise Auflagedruckes ausgebildetes erstes Druckübertragungselement 21 und zumindest ein zur Aufnahme eines von dem Kreuzbein 52 der Person ausgeübten Auflagedruckes ausgebildetes zweites Druckübertragungselement 22. Wie die Fig. 1b bis 1d darstellen, sind das erste Druckübertragungselement 21 und das zweite Druckübertragungselement 22 im Wesentlichen in einem stumpfen Winkel zueinander angeordnet. Das erste Druckübertragungselement 21 ist hierbei mittig in einem hinteren, dem Rückenelement 42 zugewandten Drittel des Sitzelements 41 angeordnet und das zweite Druckübertragungselement 22 ist ebenfalls mittig in einem unteren, dem Sitzelement 41 zugewandten Drittel des Rückenelements 42, angeordnet. Bevorzugt werden als Druckübertragungselemente 21, 22 fluidgefüllte, sehr flache Kammern eingesetzt, wobei die Kammern bevorzugt eine dem Becken 50 der Person 10 zumindest teilweise angepasste Form aufweisen. Dadurch kann eine höhere Empfindlichkeit der Druckübertragungselemente 21, 22 erreicht werden, die es erlaubt, dass Sitz- und Auflagedruckänderungen präzise erfasst werden können. Insbesondere können hierdurch bereits leichte Verschiebungen des Körperschwerpunktes erfasst werden. Unter dem Begriff Fluid ist jede Flüssigkeit oder Gas zu verstehen, die/das dem Zweck der Übertragung der Sitz- und Auflagedrücke genügt. So kann beispielsweise Wasser oder Luft als Fluid eingesetzt werden. Die Druckübertragungselemente 21, 22 können auch einfache Dehnungsmesstreifen, z. B. metallische Folien-Dehnungsmessstreifen, sein, welche von der Größe der Sitz- und Auflagedrücke abhängige, messbare Form- beziehungsweise Längenänderungen erfahren. Aus Gründen einer verbesserten Sitz- und Auflagedruckerfassung sind die Druckübertragungselemente 21, 22 sichtbar auf der oberen Oberfläche des Sitzelements 41, d.h. auf der Sitzfläche des Sitzelements 41, beziehungsweise auf der oberen Oberfläche des Rückenelements 42 angeordnet, wobei die Druckübertragungselemente 21, 22 auch nicht sichtbar in das Sitzelement 41 und/oder in die Rückenlehne 42 des Sitzsystems 40 eingebettet sein können.

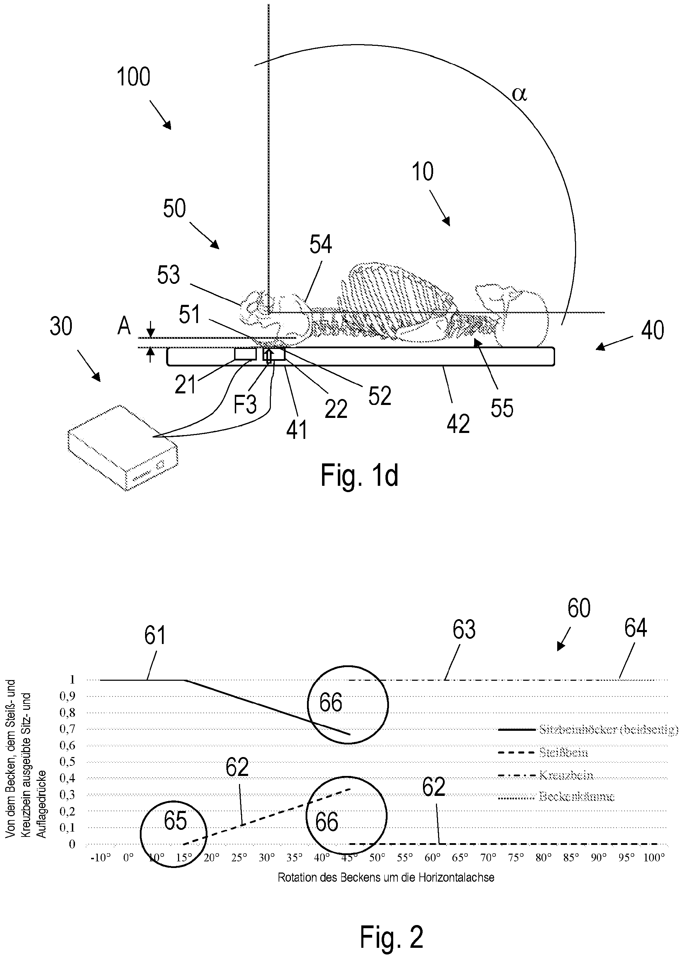

Die Fig. 1a bis 1d zeigen einzelne Lagen der Person 10 während einer Rotation dieser um die Horizontalachse, wobei die Fig. 1a die Person auf einem Sitzsystem 40 in einer ergonomisch richtigen aufrechten Sitzposition darstellt und Fig. 1d die Person 10 nach einem Ende der Rotation in einer ergonomisch richtigen horizontalen Liegeposition darstellt. Pfeile in den Fig. 1b bis 1d repräsentieren die von dem Becken 50 ausgeübten Sitz- und Auflagedrücke während der genannten Rotation, wobei F1 den von den Sitzbeinhöckern ausgebübten Sitzdruck, F2 den von dem Steißbein ausgebübten Sitzdruck, und F3 den von dem Kreuzbein ausgebübten Auflagedruck veranschaulicht.

Wie in Fig. 1a ersichtlich, werden bei einer ergonomisch richtigen aufrechten Sitzposition die Sitzbeinhöcker 53 von einem Großteil der Oberkörpergewichtskraft der Person 10 belastet, wobei kleinere Gewichtskraftanteile auch von den unteren Extremitäten der Person, nicht dargestellt, aufgenommen werden können. Bei dieser Sitzposition ist das Steißbein 51 unbelastet und weist einen Abstand A zu der Sitzfläche des Sitzelements 41 des Sitzsystems 40 auf.

Bei einer Rotation der Person 10 um einen Winkel α, Fig. 1b, reduziert sich der Abstand A, d. h. der Abstand von dem Steißbein 51 der Person 10 zu der Sitzfläche des Sitzsystems 41 verringert sich, und beträgt in dieser Lage im Wesentlichen null. Hierdurch übernimmt das Steißbein 51 einen Gewichtskraftanteil und übt zusätzlich zu den Sitzbeinhöckern 53 einen Sitzdruck auf das Sitzelement 41 aus, welcher von dem ersten Druckübertragungselement 21 erfasst wird. Diese Lage entspricht einer Kippung des Beckens 50 der Person 10 im Wesentlichen 10° um die Horizontalachse.

Ausgehend von dieser Lage, verschieben sich die Gewichtskraftanteile der Person 10 bei einer weiteren Rotation dieser so, dass sich die von den Sitzbeinhöckern 53 auf das Sitzelement 41 ausgeübten Sitzdrücke reduzieren beziehungsweise null werden, und das Steißbein 51 und das Kreuzbein 52 den Großteil der Gewichtskraft aufnehmen. Diese Lage ist in Figur 1c dargestellt und entspricht einer Kippung des Beckens 50 im Wesentlichen 45° um die Horizontalachse der Person 10. Das erste Druckübertragungselement 21 erfasst hierbei den Sitzdruck des Steißbeins 51 und das zweite Druckübertragungselement 22 erfasst den Auflagedruck des Kreuzbeins 52 der Person 10.

Bei einer weiteren Überführung der Person 10 in eine im Wesentlichen horizontale Liegeposition, Fig. 1d, verschieben sich die Gewichtskraftanteile so, dass in dieser Lage das Kreuzbein 52 der Person 10 einen maximalen Auflagedruck auf das zweite Druckübertragungselement 22 ausübt. Wie durch den Abstand A ersichtlich, übt in dieser Lage das Steißbein 51 auf das erste Druckübertragungselement 21 keinen Auflagedruck aus.

Die erfindungsgemäße Vorrichtung 100 umfasst neben den bereits erwähnten Druckübertragungselementen 21, 22 eine mit den Druckübertragungselementen 21, 22 bevorzugt über Kanäle verbundene Auswerteeinheit 30, wobei die Auswerteeinheit 30 dazu ausgebildet ist, Messsignale von den Druckübertragungselementen 21, 22 zu empfangen und auszuwerten. Das Messsignal ist eine physikalische Größe wie beispielsweise Druck, elektrischer Strom oder elektrische Spannung und ist abhängig von dem Typ der verwendeten Druckübertragungselemente 21, 22. In der oben erwähnten bevorzugten Ausführungsform, in welcher fluidgefüllte Kammern als Druckübertragungselemente 21, 22 eingesetzt werden, ist das Messsignal ein Drucksignal, welches über die Kanäle an die Auswerteeinheit 30 übertragen wird.

Fig. 2 zeigt ein Diagramm 60 mit Messreihen 61, 62, 63, 64, das Druckverläufe der während der oben beschriebenen Rotation des Beckens 50 der Person 10 auf das genannte Sitzsystem 40 ausgeübten Sitz- und Auflagedrücke darstellt. Die Skala der Ordinate des Diagramms 60 ist normalisiert dargestellt, wobei 0 kein Sitz- beziehungsweise Auflagedruck und 1 maximaler Sitz- beziehungsweise Auflagedruck bedeutet. Die Messreihe 61 zeigt den Druckverlauf der von den Sitzbeinhöckern 53 ausgeübten Sitzdrücke, wobei die Messreihe 62 den Druckverlauf des von dem Steißbein 51 ausgeübten Sitzdruckes zeigt, wobei die Messreihe 63 den Druckverlauf des von dem Kreuzbein ausgeübten Auflagedruckes zeigt und wobei die Messreihe 64 den Druckverlauf der von den Beckenkämmen 54 ausgeübten Auflagedrücke zeigt.

Die Auswerteeinheit 30 ist des Weiteren dazu ausgebildet, eine erste charakteristische Druckverteilung 65 zu bestimmen, bei der das erste Druckübertragungselement 21 während einer Rotation des Beckens 50 der Person 10 um dessen Horizontalachse erstmals mit zumindest einem Teil des Sitzdruckes des Steißbeins 51 oder das erste Druckübertragungselement 21 mit zumindest einem Teil des Auflagedruckes des Schambeins beaufschlagt wird. Die erste charakteristische Druckverteilung 65, bei der das Steißbein 51 der Person 10 erstmals mit zumindest einem Teil des Sitzdruckes beaufschlagt wird ist in Fig. 2 bei einer Neigung des Beckens 50 15° um die Horizontalachse dargestellt. Abhängig von der Nachgiebigkeit einer Untergrundstruktur, beispielsweise der Sitzfläche des Sitzelements 41 des Sitzsystems 40 und/oder aufgrund einer Abstützung der Wirbelsäule 55 an dem Rückenelement 42 und/oder pathologischen Veränderungen des Steißbeins 51, kann das Steißbein 51 bereits bei einer Neigung des Beckens 50 ab im Wesentlichen 10° um die Horizontalachse mit einem Sitzdruck beaufschlagt werden. In Bauchlage übt das Schambein der Person 10, entsprechend dem Auflagedruck des Kreuzbeins 52 der Person 10 in Rückenlage, dargestellt in der Messreihe 63 in Fig. 2, einen Auflagedruck auf das erste Druckübertragungselement 21 aus.

Die Auswerteeinheit 30 ist ferner dazu ausgebildet, eine zweite charakteristische Druckverteilung 66 zu bestimmen, bei der der auf das erste Druckübertragungselement 21 ausgeübte Sitzdruck des Steißbeins 51 einen sprunghaften Abfall aufweist und der auf das zweite Druckübertragungselement 22 ausgeübte Auflagedruck des Kreuzbeins 52 einen sprunghaften Anstieg aufweist. Diese zweite charakteristische Druckverteilung 66 ist in Figur 2 dargestellt.

Das Auswerten und das Empfangen der Messsignale von den Druckübertragungselementen 21, 22, sowie das Bestimmen der ersten charakteristischen Druckverteilung 65 und der zweiten charakteristischen Druckverteilung 66 wird durch ein Computerprogrammprodukt realisiert, welches Befehle umfasst, die bei der Ausführung des Programms durch die Auswerteeinheit 30 diese veranlassen, die zuvor erwähnten Schritte auszuführen. Das Computerprogrammprodukt ist hierbei auf einem Computerlesbaren Datenträger gespeichert.

Bevorzugt werden die charakteristischen Druckverteilungen 65, 66 durch das Bestimmen von Druckdifferenzen der von dem Becken 50, dem Steißbein 51 und Kreuzbein 52 der Person 10 auf die Druckübertragungselemente 21, 22 ausgeübten Sitz- und Auflagedrücke durch die Auswerteeinheit 30 ermittelt. Die charakteristischen Druckverteilungen 65, 66 können jedoch auch über eine Mustererkennung bestimmt werden, indem die Sitz- und Auflagedrücke mit einem vorgegebenen Druckmuster verglichen werden.

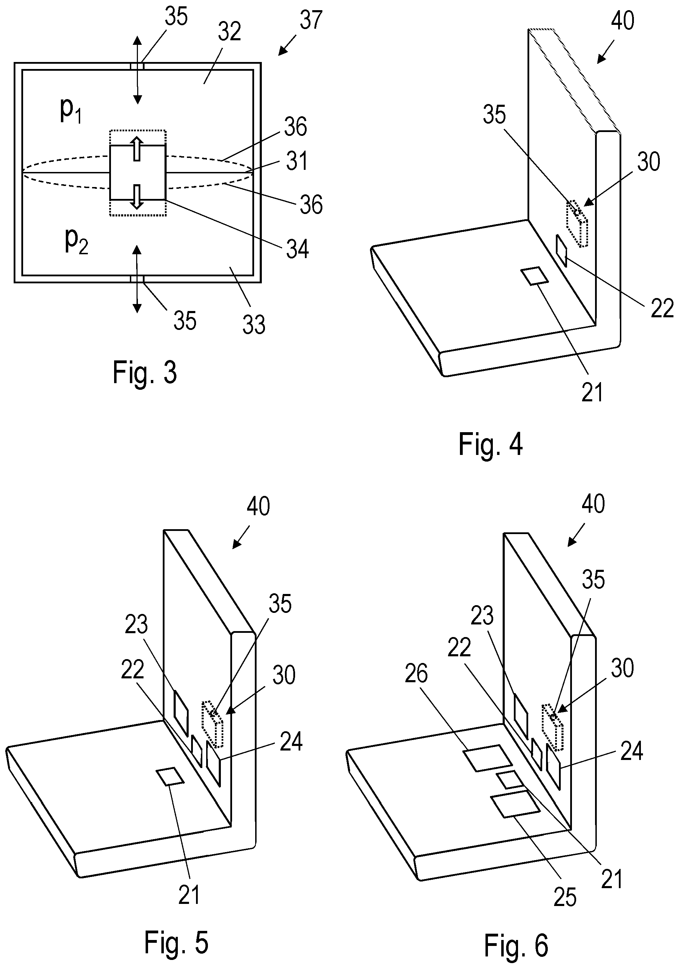

Die Fig. 3 zeigt eine bevorzugte Ausführungsform der Auswerteeinheit 30. Demnach ist die Auswerteeinheit 30 eine eine Membran 31 umfassende und mit den Druckübertragungselementen 21, 22 über Kanäle, nicht dargestellt, verbundene Membraneinheit 37, die bevorzugt die von den Druckübertragungselementen 21, 22 an die Auswerteeinheit 30 übertragenen Messsignale periodisch in einem vorgegebenen Zeitintervall auswertet. So können die Messignale beispielsweise sekündlich ausgewertet werden, um eine zuverlässige Lageerkennung des Beckens 50 der Person 10 durchführen zu können. Die Membran 31 weist ein linear-elastisches Verhalten auf und trennt die Membraneinheit 37 in zwei Kammern 32, 33, wobei eine erste Kammer 32 einen Druck p₁ aufweist und eine zweite Kammer 33 einen Druck p₂ aufweist. Die Kammern 32, 33 weisen zudem jeweils eine Öffnung 35 auf, an denen die Kanäle angeordnet sind. Die Membraneinheit 37 umfasst zudem einen an der Membran 31 angeordneten Sensor 34 und eine Rechnereinheit, welche nicht dargestellt ist. Der Sensor 34 ist bevorzugt mittig an der Membran 31 angeordnet. Die durch das Steißbein 51 und das Kreuzbein 52 der Person 10 auf das Sitzelement 41 und das Rückenelement 42 des Sitzsystems 40 ausgeübten Sitz- und Auflagedrücke bewirken eine Umverteilung des in den Druckübertragungselementen 21, 22 enthaltenen Fluides, wodurch in den Kammern 32, 33 ein Druckunterschied erzeugt wird, der zu einer ebenen Membranauslenkung 36 führt. Der an der Membran 31 angeordnete Sensor 34, ist bevorzugt ein Sensor ausgewählt aus der Gruppe bestehend aus mechanischen, elektrischen, pneumatischen oder hydraulischen Sensoren, insbesondere jedoch ein Beschleunigungssensor. Durch Erfassen einer Geschwindigkeitsänderung kann der Beschleunigungssensor die Membranauslenkung 36 detektieren und ein entsprechendes Sensorsignal an die Rechnereinheit übertragen. Die Rechnereinheit wertet im Anschluss das Sensorsignal aus, wobei das Sensorsignal bevorzugt in direkter Relation zu der Membranauslenkung 36 steht. Ist das erste Druckübertragungselement 21 mit der ersten Kammer 32 und das zweite Druckübertragungselement 22 mit der zweiten Kammer 33 der Membraneinheit 37 verbunden, herrscht bei der ersten charakteristischen Druckverteilung 65 ein Druckniveau von p₁ > p₂. Übt das Kreuzbein 52 der Person 10 einen im Vergleich zu dem von dem Steißbein 51 ausgeübten Sitzdruck größeren Auflagedruck aus, wie dies bei der zweiten charakteristischen Druckverteilung 66 gegeben ist, wird ein Druckniveau von p₂ > p₁ erreicht. Übt das Steißbein 51 einen gleich großen Sitzdruck auf erste Druckübertragungselement 21 aus, wie das Kreuzbein 52 einen Auflagedruck auf das zweite Druckübertragungselement 22 ausübt, gilt p₁ = p₂ und keine Membranauslenkung findet statt. Dies entspricht ebenfalls einem belastungsfreien Zustand der Druckübertragungselemente 21, 22, d. h. die Druckübertragungselemente 21, 22 werden nicht mit einem Sitz- und/oder Auflagedruck beaufschlagt. Der Beschleunigungssensor ist bevorzugt in einer Rechnereinheit, nicht dargestellt, integriert. Zum Beispiel kann ein Prozessor auf der Membran 31 angeordnet sein, der einen entsprechenden Beschleunigungssensor aufweist und die Sensorsignale des Beschleunigungssensors auswertet.

Figur 4 zeigt schematisch die bevorzugte Ausführungsform der gegenständlichen Erfindung in einer perspektivischen Ansicht. Die Membraneinheit 37 ist bevorzugt im Bereich der Druckübertragungselemente 21, 22 angeordnet und bevorzugt nicht sichtbar in das Rückenelement 42 des Sitzsystems 40 integriert. Die Membraneinheit 37 ist jedoch auch von dem Sitzsystem 40 losgelöst einsetzbar, wobei sich die Länge der Kanäle, die die Membraneinheit 37 mit den Druckübertragungselementen 21, 22 verbinden und in dieser Figur nicht dargestellt sind, hierbei deutlich verlängern kann. Die ebene Auslenkung 36 des hier beispielhaft angeordneten Sensors 34 in der Membraneinheit 37 erfolgt hier in der Frontalebene in longitudinale Richtung.

Die erfindungsgemäße Vorrichtung 100 kann in einer weiteren Ausführungsform ein drittes Druckübertragungselement 23 und ein viertes Druckübertragungselement 24 umfassen, dargestellt in Fig. 5, welche jeweils neben dem zweiten Druckübertragungselement 22 angeordnet und zur Aufnahme der von den Beckenkämmen 54 der Person 10 ausgeübten Auflagedrücke ausgebildet sind. Diese zusätzlichen Druckübertragungselemente 23, 24 weisen im Wesentlichen dieselbe Form auf wie das erste Druckübertragungselement 21 und das zweite Druckübertragungselement 22 und sind bevorzugt ebenfalls als fluidgefüllte Kammern ausgeführt. Durch das dritte Druckübertragungselement 23 und das vierte Druckübertragungselement 24 kann eine Membranauslenkung zusätzlich zu jener in longitudinale Richtung auch in horizontale Richtung erfolgen. Eine Membranauslenkung in horizontale Richtung entspricht dabei einer Rotation des Beckens 50 um die Longitudinalachse der Person 10. Hierfür umfasst die Membraneinheit 37 eine zweite Membran, die in den Figuren nicht dargestellt ist, welche so angeordnet ist, dass sie die Membraneinheit 37 in zwei zusätzliche Kammern, ebenfalls nicht dargestellt, d. h. insgesamt vier Kammern, teilt. Der Beschleunigungssensor ist hierbei bevorzugt an einem Kreuzungspunkt der ersten Membran und der zweiten Membran angeordnet und erfasst sowohl die ebene Membranauslenkung in longitudinale als auch in horizontale Richtung.

In einer weiteren Ausführungsform der Erfindung, dargestellt in Fig. 6, umfasst die Vorrichtung 100 ein fünftes Druckübertragungselement 25 und ein sechstes Druckübertragungselement 26, welche jeweils neben dem ersten Druckübertragungselement 21 angeordnet und zu der Aufnahme der von den Sitzbeinhöckern 53 der Person 10 ausgeübten Auflagedrücke ausgebildet sind. Das fünfte Druckübertragungselement 25 und das sechste Druckübertragungselement 26 weisen im Wesentlichen dieselbe Form auf wie das erste Druckübertragungselement 21 und das zweite Druckübertragungselement 22 und sind bevorzugt ebenfalls als fluidgefüllte Kammern ausgeführt. Bei dieser Ausführungsform ist die Membraneinheit 37 so ausgebildet, dass eine Membranauslenkung sowohl in longitudinale als auch in horizontale Richtung erfolgt. Während des Hinsetzens erfährt die Membran, aufgrund der von den Sitzbeinhöckern 53 auf das fünfte Druckübertragungselement 25 und das sechste Druckübertragungselement 26 ausgeübten maximalen Sitzdrücke, dargestellt in Fig. 2, eine maximale Membranauslenkung. Hierdurch kann die Oberkörpergewichtskraft der Person 10 bestimmt werden.

Bei dieser Ausführungsform und der zuvor genannten Ausführungsform der Erfindung, jene mit vier Druckübertragungselementen 21, 22, 23, 24, kann die Lage des Beckens 50 der Person 10 auch bei Mischbewegungen detektiert werden. Mischbewegungen können beispielsweise Bewegungen der Person 10 sein, welche gleichzeitig um die Horizontalachse und die Sagittalachse erfolgen. Diese Bewegung kann beispielsweise auftreten, wenn die Sitzbeinhöcker 53 asymmetrisch belastet werden und gleichzeitig eine Rotation des Beckens 50 der Person 10 um deren Horizontalachse erfolgt. Die Membranauslenkung erfolgt hierbei sowohl in longitudinale als auch in horizontale Richtung. Eine durch die Sitz- und Auflagedrücke des Beckens 50 verursachte Membranauslenkung in sagittale Richtung ist durch den oben beschriebenen Aufbau der Membraneinheit 37 gesperrt. Änderungen des Sensorsignals in dieser Ebene sind auf Lageänderungen des Sitzelements 41 des Sitzsystems 40 zurückzuführen, wobei dadurch ebenfalls auf die korrespondierende Lageänderung des Rückenelements 42 zurückgeschlossen werden kann. Hierbei können Rotationen des Sitzsystems 40 um die Longitudinalachse, was einer Neigung des Sitzelements 41 zur Seite entspricht, und um die Horizontalachse, was einer Neigung des Sitzelements 41 nach oben oder unten entspricht, bestimmt werden. Über eine Kräftedreieck-Berechnung kann daraus ein resultierender maximaler Sitz- und/oder Auflagedruck durch die Auswerteeinheit 30 berechnet werden. Daraus kann die Vorrichtung Sitz- und/oder Auflagedruckanteile des Beckens ermitteln, wodurch die Lage des Beckens 50 auch bei einer leichten Fehlstellung dessen bestimmbar ist.

In einer in den Figuren nicht dargestellten Ausführungsform der Erfindung umfasst die Vorrichtung 100 ein Array mit einer Vielzahl von Druckübertragungselementen, welches dazu ausgebildet ist, die Sitz- und Auflagedrücke des Beckens 50 der Person 10 in jeder Position der Person 10 auf der Vorrichtung 100 zu detektieren. Das Array mit einer Vielzahl von Druckübertragungselementen kann sowohl in dem Sitzelement 41, in dem Rückenelement 42 oder in beiden, d. h. in dem Sitzelement 41 und in dem Rückenelement 42, integriert sein. Hierdurch kann das Vorpositionieren der Person 10 auf dem Sitzsystem 40 entfallen.

In einer in den Figuren nicht dargestellten Ausführungsform, umfasst die Vorrichtung 100 ein Warnmittel, welches die Person 10 warnt, wenn die erste charakteristische Druckverteilung 65 und/oder die zweite charakteristische Druckverteilung 66 bestimmt wurde. Wurde die erste charakteristische Druckverteilung 65 und/oder die zweite charakteristische Druckverteilung 66, d. h. eine ergonomisch falsche Sitz- oder Liegeposition, durch die Auswerteeinheit 30 ermittelt, so können beispielsweise akustische Signale in Form eines von einem Lautsprecher ausgegebenen Tons oder optische Signale in Form eines von einer Warnleuchte abgegebenen Warnlichtes, die Person 10 warnen. Die Warnmittel können dabei in/an dem Sitzsystem 40 integriert/angeordnet sein oder sich von dem Sitzsystem 40 losgelöst in der Nähe dieses befinden. Die Warnmittel sind hierbei ausgewählt aus der Gruppe bestehend aus optischen, akustischen oder haptischen Warnmitteln.

Gemäß dem vorstehend beschriebenen Ausführungsbeispiel mit Flächensensoren ist bei einer Rotation des Beckens um die Horizontalachse von im Wesentlichen 45° der erfindungswesentliche Winkel gegeben, bei dem die Gewichtskraftanteile der Person auf das Sitzelement vom Steißbein und den Sitzbeinhöckern auf das Kreuzbein übergehen. Dieser erfindungswesentliche Winkel hängt aber auch vom Gewicht der jeweiligen Person und der Härte des Sitzelements ab. Dementsprechend kann dieser erfindungswesentliche Winkel in einem Winkelbereich liegen, der bei 11° beginnt, bei dem das Steißbein beginnt auf das Sitzelement zu drücken, und bei 56° endet, bei dem die Sitzbeinhöcker das Sitzelement wieder entlasten. Der erfindungswesentliche Winkel kann daher beispielsweise 35°, 40°, 43°, 45°, 47°, 50°, 53° oder 56° betragen.

Gemäß einem weiteren Ausführungsbeispiel mit einer besonders weichen Sitzauflage stellt der vorstehend für Flächensensoren beschriebene Winkelbereich mit einem erfindungswesentlichen Winkel von zumindest 11° nicht die relevante Grenze dar. Mit einer besonders weichen Sitzauflage kann tatsächlich bereits bei aufrechter Sitzposition, also bei einem erfindungswesentlichen Winkel von 0°, der Druck des Steißbeins und bei liegender Position, also bei einem erfindungsgemäßen Winkel von 90°, der Druck der Sitzbeinhöcker gemessen werden, weshalb für besonders weiche Sitzauflagen der Winkelbereich des erfindungswesentlichen Winkels von 0° bis 90° anzusetzen ist.

## Patentansprüche

1. Vorrichtung (100) zur Lagebestimmung des Beckens (50) einer Person (10), welche Vorrichtung (100) dazu ausgebildet ist die Person (10) aufzunehmen, umfassend zumindest ein zur Aufnahme eines von dem Steißbein (51) beziehungsweise Schambein der Person (10) ausgeübten Sitzdruckes beziehungsweise Auflagedruckes ausgebildetes erstes Druckübertragungselement (21) und zumindest ein zur Aufnahme eines von dem Kreuzbein (52) der Person (10) ausgeübten Auflagedruckes ausgebildetes zweites Druckübertragungselement (22) und eine mit den Druckübertragungselementen (21, 22) verbundene Auswerteeinheit (30), wobei die Auswerteeinheit (30) dazu ausgebildet ist, die folgenden Schritte auszuführen:
a) Empfangen und Auswerten von Messsignalen von den Druckübertragungselementen (21, 22);
b) Bestimmen einer ersten charakteristischen Druckverteilung (65), bei der das erste Druckübertragungselement (21) während einer Rotation des Beckens (50) der Person (10) um deren Horizontalachse erstmals mit zumindest einem Teil des Sitzdruckes des Steißbeins (51) oder das erste Druckübertragungselement (21) mit zumindest einem Teil des Auflagedruckes des Schambeins beaufschlagt wird; und
c) Bestimmen einer zweiten charakteristischen Druckverteilung (66), bei der der auf das erste Druckübertragungselement (21) ausgeübte Sitzdruck des Steißbeins (51) einen sprunghaften Abfall aufweist und der auf das zweite Druckübertragungselement (22) ausgeübte Auflagedruck des Kreuzbeins (52) einen sprunghaften Anstieg aufweist.

2. Vorrichtung (100) nach Anspruch 1, **dadurch gekennzeichnet, dass** das erste Druckübertragungselement (21) und das zweite Druckübertragungselement (22) im Wesentlichen in einem stumpfen Winkel zueinander angeordnet sind.

3. Vorrichtung (100) nach einem der vorangehenden Ansprüche, **dadurch gekennzeichnet, dass** die Auswerteeinheit (30) ferner dazu ausgebildet ist die von den Druckübertragungselementen (21, 22) an die Auswerteeinheit (30) übertragenen Messsignale periodisch in einem vorgegebenen Zeitintervall auszuwerten.

4. Vorrichtung (100) nach einem der vorangehenden Ansprüche, **dadurch gekennzeichnet, dass** die Auswerteeinheit (30) eine eine Membran (31) umfassende und mit den Druckübertragungselementen (21, 22) über Kanäle verbundene Membraneinheit (37) ist, welche einen an der Membran (31) angeordneten Sensor (34) und eine Rechnereinheit aufweist, welche zum Auswerten eines von dem Sensor (34) ausgegebenen Sensorsignals ausgebildet ist.

5. Vorrichtung (100) nach einem der vorangehenden Ansprüche, **dadurch gekennzeichnet, dass** die Vorrichtung (100) ein drittes Druckübertragungselement (23) und ein viertes Druckübertragungselement (24) umfasst, welche jeweils neben dem zweiten Druckübertragungselement (22) angeordnet und zu der Aufnahme der von den Beckenkämmen (54) der Person (10) ausgeübten Auflagedrücke ausgebildet sind.

6. Vorrichtung (100) nach Anspruch 5, **dadurch gekennzeichnet, dass** die Vorrichtung (100) ein fünftes Druckübertragungselement (25) und ein sechstes Druckübertragungselement (26) umfasst, welche jeweils neben dem ersten Druckübertragungselement (21) angeordnet und zu der Aufnahme der von den Sitzbeinhöckern (53) der Person ausgeübten Sitzdrücke ausgebildet sind.

7. Vorrichtung (100) nach einem der vorangehenden Ansprüche, **dadurch gekennzeichnet, dass** die Vorrichtung (100) ein Array mit einer Vielzahl von Druckübertragungselementen umfasst, welches dazu ausgebildet ist, die Sitz- und Auflagedrücke des Beckens (50) der Person (10) in jeder Position der Person (10) auf der Vorrichtung (100) zu detektieren.

8. Vorrichtung (100) nach Anspruch 4, **dadurch gekennzeichnet, dass** der Sensor (34) ausgewählt ist aus der Gruppe bestehend aus mechanischen, elektrischen, pneumatischen oder hydraulischen Sensoren.

9. Vorrichtung (100) nach einem der vorangehenden Ansprüche, umfassend ein Warnmittel, **dadurch gekennzeichnet, dass** das Warnmittel die Person (10) warnt, wenn die erste charakteristische Druckverteilung (65) und/oder die zweite charakteristische Druckverteilung (66) bestimmt wurde, wobei das Warnmittel ausgewählt ist aus der Gruppe bestehend aus optischen, akustischen oder haptischen Warnmitteln.

10. Computerprogrammprodukt, umfassend Befehle, die bei der Ausführung des Programms durch die Auswerteeinheit (30) der Vorrichtung nach Anspruch 1 diese veranlassen, die Schritte von Anspruch 1 mit der Vorrichtung nach Anspruch 1 auszuführen.

11. Computerlesbarer Datenträger, auf dem das Computerprogrammprodukt nach Anspruch 10 gespeichert ist.

## Claims

1. Device (100) for determining a position of the pelvis (50) of a person (10), which device (100) is designed to receive the person (10), comprising at least one first pressure transmission element (21) designed to receive a sitting pressure or support pressure exerted by the coccyx (51) or pubic bone of the person (10) and at least one second pressure transmission element (22) designed to receive a support pressure exerted by the sacrum (52) of the person (10) and an evaluation unit (30) connected to the pressure transmission elements (21, 22), wherein the evaluation unit (30) is designed to carry out the following steps:
a) Receiving and analysing measurement signals from the pressure transmission elements (21, 22);
b) Determining a first characteristic pressure distribution (65), in which the first pressure transmission element (21) is first acted upon by at least a part of the seating pressure of the coccyx (51) or the first pressure transmission element (21) is acted upon by at least a part of the support pressure of the pubic bone during a rotation of the pelvis (50) of the person (10) about its horizontal axis; and
c) Determining a second characteristic pressure distribution (66), in which the seating pressure of the coccyx (51) exerted on the first pressure transmission element (21) has an abrupt drop and the support pressure of the sacrum (52) exerted on the second pressure transmission element (22) has an abrupt rise.

2. The device (100) according to claim 1, **characterised in that** the first pressure transmission element (21) and the second pressure transmission element (22) are arranged substantially at an obtuse angle to one another.

3. The device (100) according to any one of the preceding claims, **characterised in that** the evaluation unit (30) is further designed to periodically evaluate the measurement signals transmitted by the pressure transmission elements (21, 22) to the evaluation unit (30) at a predetermined time interval.

4. The device (100) according to any one of the preceding claims, **characterised in that** the evaluation unit (30) is a diaphragm unit (37) comprising a diaphragm (31) and connected to the pressure transmission elements (21, 22) via channels, which has a sensor (34) arranged on the diaphragm (31) and a computing unit which is designed to evaluate a sensor signal output by the sensor (34).

5. The device (100) according to any one of the preceding claims, **characterised in that** the device (100) comprises a third pressure transmission element (23) and a fourth pressure transmission element (24), which are each arranged next to the second pressure transmission element (22) and are designed to absorb the contact pressures exerted by the iliac crests (54) of the person (10).

6. The device (100) according to claim 5, **characterised in that** the device (100) comprises a fifth pressure transmission element (25) and a sixth pressure transmission element (26), which are each arranged next to the first pressure transmission element (21) and are designed to absorb the seat pressures exerted by the ischial tuberosities (53) of the person.

7. The device (100) according to any one of the preceding claims, **characterised in that** the device (100) comprises an array with a plurality of pressure transmission elements, which is designed to detect the seat and support pressures of the pelvis (50) of the person (10) in any position of the person (10) on the device (100).

8. The device (100) according to claim 4, **characterised in that** the sensor (34) is selected from the group consisting of mechanical, electrical, pneumatic or hydraulic sensors.

9. The device (100) according to any one of the preceding claims, comprising a warning means, **characterised in that** the warning means warns the person (10) when the first characteristic pressure distribution (65) and/or the second characteristic pressure distribution (66) has been determined, wherein the warning means is selected from the group consisting of optical, acoustic or haptic warning means.

10. A computer program product comprising instructions which, when the program is executed by the evaluation unit (30) of the device according to claim 1, cause it to perform the steps of claim 1 with the device according to claim 1.

11. A computer-readable data carrier on which the computer program product according to claim 10 is stored.

## Revendications

1. Dispositif (100) pour déterminer la position du bassin (50) d'une personne (10), lequel dispositif (100) est conçu pour recevoir la personne (10), comprenant au moins un premier élément de transmission de pression (21) conçu pour recevoir une pression d'assise ou une pression d'appui exercée par le coccyx (51) ou le pubis de la personne (10) et au moins un deuxième élément de transmission de pression (22) conçu pour recevoir une pression d'appui exercée par le sacrum (52) de la personne (10), et une unité d'évaluation (30) reliée aux éléments de transmission de pression (21, 22), l'unité d'évaluation (30) étant conçue pour exécuter les étapes suivantes :
a) recevoir et évaluer des signaux de mesure provenant des éléments de transmission de pression (21, 22) ;
b) déterminer une première répartition de pression caractéristique (65), dans laquelle le premier élément de transmission de pression (21) est soumis pour la première fois à au moins une partie de la pression d'assise du coccyx (51) ou le premier élément de transmission de pression (21) est soumis à au moins une partie de la pression d'appui du pubis pendant une rotation du bassin (50) de la personne (10) autour de son axe horizontal ; et
c) déterminer une deuxième distribution de pression caractéristique (66), dans laquelle la pression d'assise du coccyx (51) exercée sur le premier élément de transmission de pression (21) présente une chute brutale et la pression d'appui du sacrum (52) exercée sur le deuxième élément de transmission de pression (22) présente une augmentation brutale.

2. Le dispositif (100) selon la revendication 1, **caractérisé en ce que** le premier élément de transmission de pression (21) et le deuxième élément de transmission de pression (22) sont disposés sensiblement selon un angle obtus l'un par rapport à l'autre.

3. Le Dispositif (100) selon l'une des revendications précédentes, **caractérisé en ce que** l'unité d'évaluation (30) est en outre conçue pour évaluer périodiquement, dans un intervalle de temps prédéterminé, les signaux de mesure transmis par les éléments de transmission de pression (21, 22) à l'unité d'évaluation (30).

4. Le dispositif (100) selon l'une des revendications précédentes, **caractérisé en ce que** l'unité d'évaluation (30) est une unité à membrane (37) comprenant une membrane (31) et reliée aux éléments de transmission de pression (21, 22) par des canaux, qui présente un capteur (34) disposé sur la membrane (31) et une unité de calcul qui est conçue pour évaluer un signal de capteur émis par le capteur (34).

5. Le dispositif (100) selon l'une quelconque des revendications précédentes, **caractérisé en ce que** le dispositif (100) comprend un troisième élément de transmission de pression (23) et un quatrième élément de transmission de pression (24), qui sont disposés respectivement à côté du deuxième élément de transmission de pression (22) et sont adaptés pour recevoir les pressions d'appui exercées par les crêtes iliaques (54) de la personne (10).

6. Le dispositif (100) selon la revendication 5, **caractérisé en ce que** le dispositif (100) comprend un cinquième élément de transmission de pression (25) et un sixième élément de transmission de pression (26), qui sont disposés respectivement à côté du premier élément de transmission de pression (21) et sont adaptés pour recevoir les pressions d'assise exercées par les ischions (53) de la personne.

7. Le dispositif (100) selon l'une quelconque des revendications précédentes, **caractérisé en ce que** le dispositif (100) comprend un réseau comportant une pluralité d'éléments de transmission de pression, qui est conçu pour détecter les pressions d'assise et d'appui du bassin (50) de la personne (10) dans chaque position de la personne (10) sur le dispositif (100).

8. Le dispositif (100) selon la revendication 4, **caractérisé en ce que** le capteur (34) est choisi dans le groupe constitué par les capteurs mécaniques, électriques, pneumatiques ou hydrauliques.

9. Le dispositif (100) selon l'une quelconque des revendications précédentes, comprenant un moyen d'avertissement, **caractérisé en ce que** le moyen d'avertissement avertit la personne (10) lorsque la première distribution caractéristique de pression (65) et/ou la deuxième distribution caractéristique de pression (66) a été déterminée, le moyen d'avertissement étant choisi dans le groupe constitué par les moyens d'avertissement optiques, acoustiques ou haptiques.

10. Produit programme d'ordinateur comprenant des instructions qui, lorsque le programme est exécuté par l'unité d'évaluation (30) du dispositif selon la revendication 1, amènent celle-ci à exécuter les étapes de la revendication 1 avec le dispositif selon la revendication 1.

11. Support de données lisible par ordinateur, sur lequel est enregistré le produit programme d'ordinateur selon la revendication 10.
